(19) Europäisches Patentamt
European Patent Office
Office européen des brevets

(11) **EP 2 723 760 B1**

(12) **EUROPÄISCHE PATENTSCHRIFT**

(45) Veröffentlichungstag und Bekanntmachung des Hinweises auf die Patenterteilung:
**22.01.2020 Patentblatt 2020/04**

(21) Anmeldenummer: **12719269.8**

(22) Anmeldetag: **28.04.2012**

(51) Int Cl.:
*C07K 1/34* (2006.01)    *B01D 61/14* (2006.01)
*B01D 61/16* (2006.01)    *C07K 1/36* (2006.01)

(86) Internationale Anmeldenummer:
**PCT/EP2012/001840**

(87) Internationale Veröffentlichungsnummer:
**WO 2012/175156 (27.12.2012 Gazette 2012/52)**

(54) **VERFAHREN ZUR ABTRENNUNG VON BIOPOLYMER-AGGREGATEN UND VIREN AUS EINEM FLUID**

METHOD FOR SEPARATING BIOPOLYMER AGGREGATES AND VIRUSES FROM A FLUID

PROCÉDÉ POUR LA SÉPARATION D'AGRÉGATS DE BIOPOLYMÈRE ET DE VIRUS D'UN FLUIDE

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **24.06.2011 DE 102011105525**

(43) Veröffentlichungstag der Anmeldung:
**30.04.2014 Patentblatt 2014/18**

(73) Patentinhaber: **Sartorius Stedim Biotech GmbH 37079 Göttingen (DE)**

(72) Erfinder:
• **THOM, Volkmar,
37083 Göttingen (DE)**
• **HANSMANN, Björn
37077 Göttingen (DE)**

(74) Vertreter: **Stehl, Astrid
Sartorius Stedim Biotech GmbH
Intellectual Property Management
August-Spindler-Straße 11
37079 Göttingen (DE)**

(56) Entgegenhaltungen:
EP-A1- 1 624 950    WO-A1-2010/098867
DE-A1- 10 114 537    JP-A- 2005 145 852
US-A1- 2009 326 211    US-B2- 7 118 675
US-B2- 7 465 397

• **SAXENA A ET AL: "Membrane-based techniques for the separation and purification of proteins: An overview", ADVANCES IN COLLOID AND INTERFACE SCIENCE, ELSEVIER, NL, Bd. 145, Nr. 1-2, 30. Januar 2009 (2009-01-30), Seiten 1-22, XP025716991, ISSN: 0001-8686, DOI: 10.1016/J.CIS.2008.07.004 [gefunden am 2008-08-05]**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Abtrennung von Biopolymer-Aggregaten und Viren aus einem Fluid.

Biopolymer-Aggregate, wie z. B. Aggregate aus Proteinen, und Viren sind häufig unerwünschte Kontaminanten in Fluiden, wie in Protein- und Peptidlösungen, die durch biotechnologische Verfahren gewonnen werden. Die staatlichen Gesundheitsbehörden schreiben die Entfernung dieser Kontaminanten aus dem Fluid vor, bevor dieses zum therapeutischen Einsatz kommt.

Proteinlösungen, wie beispielsweise Immunoglobuline in Blutplasma oder Lösungen, die rekombinante Proteine oder natürliche Proteine umfassen, enthalten im Allgemeinen einen nachweisbaren Anteil an Dimeren und höheren Aggregaten dieser Proteine. Vor der parenteralen Verabreichung an Patienten ist eine Abreicherung der Aggregate erforderlich. Generell wird ein Aggregatgehalt von in der Summe weniger als 1 % bezogen auf den Gesamtproteingehalt der Lösung angestrebt. Die Entfernung dieser Aggregate über Größenausschlussfiltration ist technisch nicht vorteilhaft, da es dabei zu einer Verblockung der verwendeten Filter kommt. Alternativ wurden Verfahren etabliert, die über selektive Affinitätsbindung an chromatographische Säulen, wie Ionentauscher, Verunreinigungen, z. B. Aggregate von Biopolymeren, entfernen können.

Für die Abreicherung von Viren als Kontaminanten aus therapeutisch angewandten Proteinlösungen ist die Größenausschlussfiltration als ein effektiver Prozeß bekannt, der auf alle Arten von Viren und unter einer großen Bandbreite an Bedingungen zuverlässig anwendbar ist. Die Filtration von Proteinlösungen zur Abreicherung von kleinen, nicht umhüllten Viren ist ein von den staatlichen Gesundheitsbehörden anerkannter, robuster Schritt zur Reduzierung der Virenkonzentration in Proteinlösungen, die aus biologischen Quellen stammen. Dabei sind in einem Herstellprozeß mindestens zwei verschieden wirkende, robuste Verfahren zur Abreicherung von Viren erforderlich.

An die effektive Eliminierung von Viren aus Proteinlösungen werden die für Filtrationen typischen Anforderungen gestellt: Die Selektivität der Trennung sollte zu einer Reduzierung des Titers, d. h. der Virenkonzentration, um mindestens den Faktor $10^3$ führen. Die Permeabilität von Wasser der für die Abtrennung der Viren verwendeten Filtrationsmembran sollte maximal sein. Die Tendenz zur Verblockung, d. h. die Reduzierung der Flußleistung durch eine Obstruktion durch Begleitkomponenten oder der Verschluß von Poren der Filtrationsmembran, sollte dagegen minimiert sein. Durch die gleichzeitig erforderliche Passage der therapeutischen Proteine als Zielverbindungen, die im hydrodynamischen Radius in einer ähnlichen Größenordnung liegen wie die zurückzuhaltenden Viren, ist die Herstellung von geeigneten sogenannten "Parvovirenfiltern" verglichen mit Mikrofiltrationsmembranen für andere Anwendungen aufwendig. Durch die geringe Größendifferenz zwischen Proteinen und Viren ergibt sich eine besondere Empfindlichkeit der Parvovirenfilter gegenüber Verblockung und ein im Vergleich zu Mikrofiltern hoher Anspruch an die Reinheit der zu filtrierenden Lösung.

Die Parvovirenfiltration wird wegen dieser hohen Anforderungen an die Produktreinheit relativ spät in der Prozeßabfolge zur Herstellung von rekombinanten Proteinen oder Blutplasmaprodukten durchgeführt. Üblicherweise gehen der Filtration chromatographische Trenn- und Reinigungsprozesse voraus. Von hoher Relevanz für die Virenfiltration ist die Anforderung, daß aus den verwendeten Filtrationsmedien keine extrahierbaren Bestandteile ("Leachables") oder Partikel in das Filtrat freigesetzt werden. Die Freisetzung derartiger Kontaminanten tritt bevorzugt bei Verwendung von kieselgurhaltigen Tiefenfiltern auf.

US 7,118,675 B2 offenbart ein Verfahren zur selektiven Entfernung von Protein-Aggregaten und Viren aus einer Protein-Lösung, bei welchem in einem ersten Schritt die Proteinlösung zur Retention der Aggregate durch eine oder mehrere Lagen einer geladenen oder oberflächenmodifizierten mikroporösen Membran filtriert wird. Das von den Protein-Aggregaten befreite Filtrat wird in einem zweiten Schritt durch eine oder mehrere Ultrafiltrationsmembranen filtriert, wobei der Virengehalt um mindestens drei Zehnerpotenzen reduziert wird. Dieses Verfahren, welches sich prinzipiell bewährt hat, erfordert in Abhängigkeit von der zu verarbeitenden Proteinlösung und der abzutrennenden Aggregate eine individuelle Anpassung des pH-Werts und des Leitfähigkeitswerts der Proteinlösung. Diese vorgenannten Parameter beeinflussen ihrerseits die optimale Wahl einer geeigneten geladenen bzw. oberflächenmodifizierten Membran für den ersten Schritt des Verfahrens. Alternativ können im ersten Schritt zusätzlich zu den mikroporösen Membranen eine oder mehrere Lagen eines Tiefenfilters eingesetzt werden.

Dieses Optimierungsproblem wurde bereits von A. Brown et. al. in "Increasing Parvovirus Filter Throughput of Monoclonal Antibodies Using Ion Exchange Membrane Adsorptive Pre-Filtration", Bioseparations and Downstream Processing, Biotechnology and Bioengineering, DOI 10.1002/bit.22729, 2010 Wiley Periodicals, Inc. erkannt. Geladene Membranen können zur Abtrennung von Biopolymer-Aggregaten aus virenenthaltenden Lösungen nur in einem engen pH-Bereich und bei geringen Salzkonzentrationen effektiv eingesetzt werden, weil bei diesen Membranen bei hohen Salzkonzentrationen eine unerwünschte Konkurrenz zwischen der Bindung von Ionen des Salzes und den Antikörpern als Zielproteinen auftritt.

A. Brown et. al. beschreiben die Verwendung von geladenen Membran-Vorfiltern zur Erhöhung der Filtrationskapazität von nachgeschalteten Parvovirenfiltern Viresolve®. Die Vorfilter entfernen hierbei Aggregate von monoklonalen Antikörpern, wobei die Monomere des Antikörpers das Zielprodukt sind. Für kationenaustauschende Membranen mit Sulfonsäureliganden (Mustang® S) als Virus-Vorfilter wurden im

pH-Bereich zwischen 4 und 7 und bei Leitfähigkeitswerten zwischen 3 mS/cm und 25 mS/cm die Filtrationskapazitäten eines Parvovirenfilters bestimmt. Die erzielten Filtrationskapazitäten, definiert als $V_{50}$ (Filtrationsvolumen bei einem Permeabilitätsabfall von 50 % gegenüber dem Filtrationsbeginn), zeigen eine starke Abhängigkeit von pH- und Leitfähigkeitswert, die zusätzlich durch den eingesetzten monoklonalen Antikörper beeinflußt wird. Die Abhängigkeit vom pH-Wert wird durch die Änderung der Nettoladung der Proteine und der Aggregate erklärt. Die Abhängigkeit von der Leitfähigkeit wird einer Abschirmung der geladenen Gruppen des Vorfilters durch Salze zugeschrieben. Ein Optimum ("Sweet spot") ergibt sich bei dem verwendeten monoklonalen Antikörper aus "Chinese-Hamster-Ovary"-Zellen im pH-Bereich von 5 bis 6 und bei einer Leitfähigkeit zwischen 5 mS/cm und 12 mS/cm. Die Unterschiede zwischen den o. g. optimierten Bedingungen des "Sweet Spot" und anderen suboptimalen Bereichen von pH-Wert und Leitfähigkeit unterscheiden sich voneinander bis um einen Faktor 9 bezüglich $V_{50}$.

Diese optimierten Bedingungen für den "Sweet Spot" sind bei der Verwendung von geladenen Membranen zur Aggregatentfernung für jedes Protein individuell zu ermitteln, wobei diese Optimierung einen hohen Aufwand zur erfolgreichen Anwendung dieses Verfahrens bedeutet.

EP 1 624 950 B1 offenbart ein Verfahren zur selektiven Entfernung von Protein-Aggregaten und Viren aus einer Protein-Lösung, bei welchem in einem ersten Schritt die Aggregate durch ein Faserpad und Kieselgur zurückgehalten werden, während im nachfolgenden zweiten Schritt die Viren durch mindestens eine Ultrafiltrationsmembran zurückgehalten werden, wobei der Virengehalt um mindestens drei Zehnerpotenzen reduziert wird. Das Faserpad kann eine oder mehrere Schichten von mikroporösen Membranen aus z. B. Polyamid, regenerierter Cellulose, Poly(ether)sulfon, Polyimid oder PVDF enthalten, die ihrerseits zur verbesserten Aggregatrückhaltung mit geladenen Partikeln gefüllt sind, welche aus US 5,531,899 oder 5,093,197 bekannt sind. Als geladene Partikel werden z. B. Partikel auf Basis von Diphonix-Kationenaustauscherharzen oder Partikel mit Silanolgruppen auf ihrer Oberfläche in Polyolefinmembranen verwendet. Alternativ kann die als Vorfilter zu verwendende Membran mit Kieselgelpartikeln gefüllt sein.

Bei der Verwendung von Membranen, die mit geladenen Partikeln gefüllt sind, ergibt sich hierbei das vorgenannte Optimierungsproblem bezüglich pH- und Leitfähigkeitswert der zu verarbeitenden Proteinlösung.

Bezüglich einer unerwünschten Freisetzung von Partikeln in das die Zielproteine enthaltende Filtrat kann der Einsatz von Kieselgur im ersten Schritt des Verfahrens problematisch sein.

Ein weiteres Verfahren, bei welchem als Vorfilter für den nachgeschalteten Virenfilter ebenfalls eine geladene Membran eingesetzt wird, offenbart US 7,465,397 B2. Bei diesem Verfahren erfolgt die Abtrennung von Viren und Proteinaggregaten aus einer Proteinlösung in mindestens zwei Schritten, bei welchem in der ersten Filtrationsstufe oberflächen- bzw. durch Ladung modifizierte Polyamidmembranen eingesetzt werden, während das nachgeschaltete Ultrafiltrationsmodul zur Virenentfernung Polyamidmembranen umfaßt, deren Oberfläche nicht modifiziert ist.

US 2009/0326211 A1 offenbart ein Verfahren zur selektiven Abtrennung von großen mikrobiologischen Partikeln, wie Algen, Pilzen, Amöben oder anorganischen Partikeln aus einer Wasserprobe, welche Mikroorganismen, wie Legionella pneumophila oder Viren enthält. Die Abtrennung erfolgt hier im Unterschied zu den anderen bereits genannten Dokumenten nach dem Prinzip der Größenausschlussfiltration und kann weitgehend unabhängig vom Material des Filters in gleicher Weise durchgeführt werden. Eine Abtrennung nach dem Prinzip der Größenausschlussfiltration löst allerdings ebenfalls nicht das bereits für die anderen Dokumente diskutierte Optimierungsproblem bezüglich pH- und Leitfähigkeitswert der zu verarbeitenden Proteinlösung.

Im ersten Schritt des Verfahrens nach US 2009/0326211 A1 werden die Partikel durch einen Vorfilter bestehend aus einem Stahlsieb, Glasfasern, Polypropen, Polyethersulfon, Nylon, PVC, PTFE, Polyester, Polycarbonat oder Polyester zurückgehalten, während die Mikroorganismen den Vorfilter passieren. Das die Mikroorganismen enthaltende Filtrat wird anschließend einem Hauptfilter mit geringerer Porengröße zugeführt, durch welchen die Mikroorganismen zurückgehalten werden. Anschließend können die Mikroorganismen zur Analyse einer Lyse auf dem Hauptfilter unterzogen werden. Das Material des Hauptfilters ist aus der Gruppe der Materialien ausgewählt, die auch für den Vorfilter verwendet werden.

WO 2010/098867 A1 offenbart ein zweistufiges Verfahren zur selektiven Abreicherung von Protein-Aggregaten und Viren zur Herstellung einer Lösung von Protein-Monomeren. In einem ersten Filtrationsschritt werden mit mikroporösen, beschichteten Membranen, welche negativ geladen sind, selektiv die ProteinAggregate aus der Lösung entfernt. In einem zweiten Filtrationsschritt werden die Viren durch einen Hauptfilter, d. h. durch eine Ultrafiltrationsmembran, mit einem LRV-Wert von mindestens 3 zurückgehalten.

JP 2005-145852 A offenbart ein Verfahren zur Entfernung von Antikörper-Agglomeraten über eine Polyamidmembran bei einem pH-Wert von beispielsweise 6 bis 8.

Aufgabe der vorliegenden Erfindung ist die Bereitstellung eines Verfahrens, welches die vorgenannten Nachteile des Stands der Technik überwindet und welches auf einfache und kostengünstige Weise die selektive Entfernung von Biopolymer-Aggregaten und Parvoviren aus biotechnologischen Fluiden erlaubt. Dieses Verfahren soll die Verwendung von aufwendig herstellbaren, geladenen oder oberflächenmodifizierten Membranen als Virus-Vorfiltern und von Materialien, die Partikel in das Filtrat freisetzen können, vermeiden. Darüberhinaus soll das bereitzustellende Verfahren die zuverlässige selektive Abtrennung dieser Aggregate und Parvoviren über einen weiten Bereich für den pH- und Leitfähigkeitswert der zu verarbeitenden Proteinlösung ermöglichen.

Diese Aufgabe wird durch die in den Ansprüchen gekennzeichneten Ausführungsformen der vorliegenden Erfindung gelöst.

Insbesondere wird erfindungsgemäß ein Verfahren zur Abtrennung von Biopolymer-Aggregaten und Parvoviren aus einem Fluid bereitgestellt, welches die folgenden Schritte umfasst:

(a) Filtrieren des Fluids, welches die Biopolymer-Aggregate und Parvoviren enthält, durch einen porösen, Polyamid umfassenden Formkörper, dessen innere und äußere Oberflächen die gleichen chemischen und physikalischen Eigenschaften aufweisen wie die von den Oberflächen eingeschlossene Matrix des Formkörpers, wobei selektiv die Biopolymer-Aggregate adsorptiv aus dem Fluid abgereichert werden, während die Parvoviren durch den Formkörper permeieren; und

(b) Filtrieren des Fluids, das durch Filtration im Schritt (a) erhalten wurde, durch mindestens eine Membran mit einem Molekulargewichtsausschlußbereich von 100 bis 1.000 kD, wobei der Gehalt an Parvoviren in dem Fluid gegenüber dem Gehalt an Parvoviren vor Durchführen von Schritt (a) um mindestens 99,9 % reduziert wird;

wobei der poröse, Polyamid umfassende Formkörper eine mikroporöse Membran ist und aus einem aliphatischen und/oder aromatischen Polyamid mit höchstens fünf verschiedenen Wiederholungseinheiten besteht, wobei die Membran, die im Schritt (b) verwendet wird, aus Polyethersulfon, Polysulfon oder Gemischen davon besteht, wobei die Biopolymer-Aggregate aus der Gruppe der Dimere, Trimere oder Multimere von Peptiden, Proteinen, Nukleinsäuren oder Gemischen davon ausgewählt sind und wobei der pH-Wert des Fluids während der Schritte (a) und (b) zwischen 5 und 9 beträgt.

Der Begriff "Parvovirus" umfasst erfindungsgemäß kleine, unbehüllte Viren wie beispielsweise PPV oder MVM.

Der Begriff "Fluid" unterliegt erfindungsgemäß keiner Einschränkung. Gemäß einer bevorzugten Ausführungsform der vorliegenden Erfindung umfasst das Fluid ein Blutplasmaprodukt, eine aus einer Zellkultur gewonnene Proteinlösung, eine aus einer Extraktion von tierischen oder pflanzlichen Produkten gewonnene Proteinlösung oder eine aus Mikroorganismen gewonnene Proteinlösung.

Im Schritt (a) des erfindungsgemäßen Verfahrens erfolgt die selektive Entfernung der Biopolymer-Aggregate durch Filtration durch einen porösen, Polyamid umfassenden Formkörper, welcher eine mikroporöse Membran ist, während die Parvoviren und Zielverbindungen durch den Formkörper permeieren. Erfindungsgemäß wird unter "selektiver Entfernung von Biopolymer-Aggregaten" verstanden, dass der Biopolymer-Aggregatgehalt des Fluids nach der Filtration gegenüber dem Biopolymer-Ausgangsaggregatgehalt reduziert ist. Durch diese selektive Entfernung der Biopolymer-Aggregate im ersten Schritt des erfindungsgemäßen Verfahrens kann so vorteilhafterweise eine Verblockung der Poren virusretentiver Membranen im Schritt (b) des Verfahrens vermieden werden. Erfindungsgemäß handelt es bei dem Filtermaterial im Schritt (a) um eine native Oberfläche eines Polyamids in poröser Form. Nativ bedeutet in diesem Zusammenhang, dass die inneren und äußeren Oberflächen des Polyamid umfassenden Formkörpers die gleichen chemischen und physikalischen Eigenschaften aufweisen wie die Matrix (das Grundmaterial).

Unter Polyamid wird erfindungsgemäß ein Polymer aus sich wiederholenden Einheiten verstanden, die durch Amid-Funktionen aneinander kovalent gebunden sind. Die Monomereinheiten können aliphatisch und/oder aromatisch sein und über Ringöffnung oder Polykondensation miteinander verbunden sein. Erfindungsgemäß besteht der poröse, Polyamid umfassende Formkörper aus einem aliphatischen und/oder aromatischen Polyamid mit höchstens fünf verschiedenen Wiederholungseinheiten (Monomeren). Unter den vorgenannten Monomeren sind $\varepsilon$-Caprolactam, 1,6-Hexandisäure und 1,6-Hexandiamin bzw. Sebacinsäure besonders bevorzugt. Gemäß einer bevorzugten Ausführungsform wird ein Polyamid verwendet, das nur aus aliphatischen Monomereinheiten besteht. Gemäß einer besonders bevorzugten Ausführungsform des erfindungsgemäßen Verfahrens wird Polyamid 6 und/oder Polyamid 6.6 als Polyamid verwendet.

Der poröse, Polyamid umfassende Formkörper ist erfindungsgemäß eine mikroporöse Membran. Gemäß einer bevorzugten Ausführungsform der vorliegenden Erfindung weist die Polyamid-Oberfläche eine Porengröße kleiner 0,5 μm auf.

Im Schritt (b) des erfindungsgemäßen Verfahrens erfolgt eine Filtration des Fluids aus Schritt (a) durch mindestens eine virusretentive Membran. Der Membranfilter gilt in diesem Zusammenhang als virusretentiv, wenn der Membranfilter eine Abreicherung von kleinen Viren, wie z.B. dem PPV (*plum pox virus,* Scharka-Virus), und Modellsystemen, wie dem Bakteriophagen PP7, von mindestens 99,9 % erreicht. Gemäß einer bevorzugten Ausführungsform der vorliegenden Erfindung wird zudem eine Abreicherung umhüllter Viren von mindestens 99,99 % erreicht. In diesem Zusammenhang wird der Fachmann leicht eine aus dem Stand der Technik bekannte, geeignete virusretentive Membran auswählen, die diesem Standard entspricht und zudem die Zielmoleküle permeieren lässt.

Die Membran weist erfindungsgemäß einen Molekulargewichtsausschlussbereich von 100 bis 1.000 kD auf.

Der Molekulargewichtsausschlussbereich, engl. "molecular weight cut off (MWCO)" einer Membran bezeichnet in diesem Zusammenhang das nominelle Molekulargewicht von Molekülen und Partikeln, welche die Membran zu 90 % passieren können. Dazu wird der Fachmann in ihm bekannter Weise die Molekulargewichtsverteilung eines Filtrats der zu untersuchenden Membran von einer Mischung von Dextranen verschiedener Molmasse mit dem Unfiltrat in einer Gelpermeationschromatographie vergleichen, wobei der Fluss durch die Membran auf 0,0001 cm/s eingestellt wird.

Erfindungsgemäß besteht die Membran, die im Schritt (b) verwendet wird, aus Polyethersulfon, Polysulfon oder Gemischen davon.

Erfindungsgemäß beträgt der pH-Wert des Fluids während der Schritte (a) und (b) zwischen 5 und 9. Durch die Verwendung einer porösen, Polyamid umfassenden Membran hat das erfindungsgemäße Verfahren gegenüber der Verwendung von bekannten chromatographischen Säulen besondere Vorteile. Durch selektive Entfernung der Biopolymer-Aggregate im ersten Schritt des erfindungsgemäßen Verfahrens kann vorteilhafterweise eine Verblockung der Poren virusretentiver Membranen im Schritt (b) des Verfahrens vermieden werden. Überraschenderweise wurde bei der Filtration eines Fluids durch einen aus einer mikroporösen Membran bestehenden, Polyamid umfassenden Formkörper mit einer nativen Oberfläche vor der Filtration über eine virusretentive Membran eine deutliche Erhöhung der Filtrationskapazität der virusretentiven Membran gegenüber der Verwendung von Vorfiltern aus anderen Materialien, wie z.B. Polythersulfon und insbesondere von Membranen, deren Oberflächen geladen oder gegenüber ihrer Matrix chemisch oder physikalisch modifiziert sind, gefunden. Die Erhöhung der Filtrationskapazität von Virenfiltern funktioniert unabhängig von der Nettoladung der Proteinaggregate und das Verhältnis zwischen Volumen der Proteinlösung und der Oberfläche beeinflusst die Filtrationskapazität proportional. Darüber hinaus können die Oberflächeneffekte des Polyamids mit einer Größenausschlusswirkung kombiniert werden. Vorteilhafterweise ist die Erhöhung der Filtrationskapazität unabhängig von dem pH-Wert des Fluids im Bereich von pH = 5 bis 9, was das erfindungsgemäße Verfahren von den dem Fachmann bekannten Verfahren mit geladenen oder oberflächenmodifizierten Membranen unterscheidet. Darüber hinaus weist die poröse Oberfläche des Polyamid umfassenden, aus einer mikroporösen Membran bestehenden Formkörpers vorteilhafterweise nur eine geringe Abgabe von organischem Kohlenstoff und Partikeln auf im Vergleich zu typischen Tiefenfiltermaterialien, die für die Entfernung von Biopolymer-Aggregaten beschrieben werden und sich deshalb in typischen Prozessen zur Virusabreicherung aus Proteinlösungen nur bedingt eignen. Zudem sind die für das erfindungsgemäße Verfahren verwendeten porösen Polyamid-Oberflächen, erfindungsgemäß in Form von mikroporösen Membranen, gegenüber klassischen Chromatographiemedien deutlich günstiger zugänglich. Dementsprechend ist die Trennung über das erfindungsgemäße Verfahren mit dem gleichen Volumen an Medium vorteilhafterweise nicht nur deutlich selektiver und schneller als mit den bisher bekannten Verfahren möglich, sondern auch noch deutlich kostengünstiger.

Die vorliegende Erfindung wird anhand der folgenden, nicht einschränkenden Beispiele näher erläutert.

**Beispiele**

In den folgenden Beispielen ist die Filtrationskapazität $V_{max}$ der asymptotische Wert für das Filtrationsvolumen bei 100 % Verblockung des Filters, erhalten als Kehrwert der Geradensteigung aus einer Auftragung vom Quotienten $t/V$ gegen $t$ bei Filtration bei konstantem Druck wie in Ho et. al., Journal of Colloid and Interface Science 232, 389-399 (2000), beschrieben (Gleichung 1):

$$\frac{t}{V} = \frac{1}{V_{max}} \cdot t + \frac{1}{Q_0} \qquad \text{(Gleichung 1)}$$

Dabei bezeichnet $t$ die Filtrationszeit, $V$ das Filtrationsvolumen, $V_{max}$ die Filtrationskapazität und Qo den Anfangsfluss bei der Filtration mit konstantem Druck.

Beispiel 1: Filtration einer Proteinlösung eines monoklonalen Antikörpers aus der Zelllinie CHO dg44 ST1-C6

Der monoklonale Antikörper in der Proteinlösung stammt aus der Zelllinie CHO dg44 ST1-C6 und wurde durch folgendes Verfahren bereitgestellt:
Zunächst wurde eine Zellkultur angelegt, in der das Zielprotein hergestellt wurde. Die Kultivierung fand unter üblichen, dem Fachmann bekannten Bedingungen statt, unter Verwendung eines Biostat® CultiBag RM Einwegbioreaktors (Sartorius Stedim Biotech GmbH) mit einem Kultivierungsvolumen von 25 L und einer Animpfzelldichte von $5 \times 10^5$ Zellen/mL und dem Nährmedium Pro CHO5 (Lonza BE12-766P).

Nach dem Erreichen der maximalen Zelldichte nach 9 Tagen wurden die Zellen geerntet und die Aufreinigung durchgeführt. In einem typischen Verfahren wurde die Zellsuspension zunächst über Tiefenfilterschichten und Partikelfilter mit einer Porengröße von 0,2 μm filtriert. In chromatographischen Verfahren, wie Protein-A-Affinitätschromatogra-

phie und Ionenaustauschchromatographie, wurde der Antikörper aus der zellfreien Lösung isoliert.

Zur Herstellung der Proteinlösung für die Filtration wurde die aufgereinigte Lösung nach den chromatographischen Schritten einer Ultrafiltration/Diafiltration unterzogen. Dazu wurde eine Sartocon Slice, Hydrosart® 30kD MWCO (3051445901E--SW) Filtrationskassette mit einer Filtrationsfläche von 0,1 m² und einem Molekulargewichtstrenngrenze von 30 kD verwendet. Nach diesem Schritt zur Veränderung der Pufferzusammensetzung und Proteinkonzentration wurde die Lösung des Antikörpers mit einer Konzentration von 10 g/L in Phosphat-Puffer, pH 6,6 (Sigma Aldrich, Katalog Nr. P8165), und 10 mM EDTA (Sigma Aldrich, Katalog Nr.:E6758) erhalten.

Die Proteinlösung des monoklonalen Antikörpers wurde im Schritt (a) über eine poröse Polyamid-Oberfläche aus Polyamid 6 in Form einer mikroporösen Membran (Porengröße 0,2 μm, äußere Fläche 17,5 cm²) geleitet. Anschließend wurde das Filtrat aus Schritt (a) im Schritt (b) über eine Lage einer virusretentiven Membran, Virosart® CPV Minisart (Polyethersulfonmembran, Porengröße 0,02 μm, Fläche 5 cm², Sartorius Stedim Biotech GmbH), bei 1 bar filtriert.

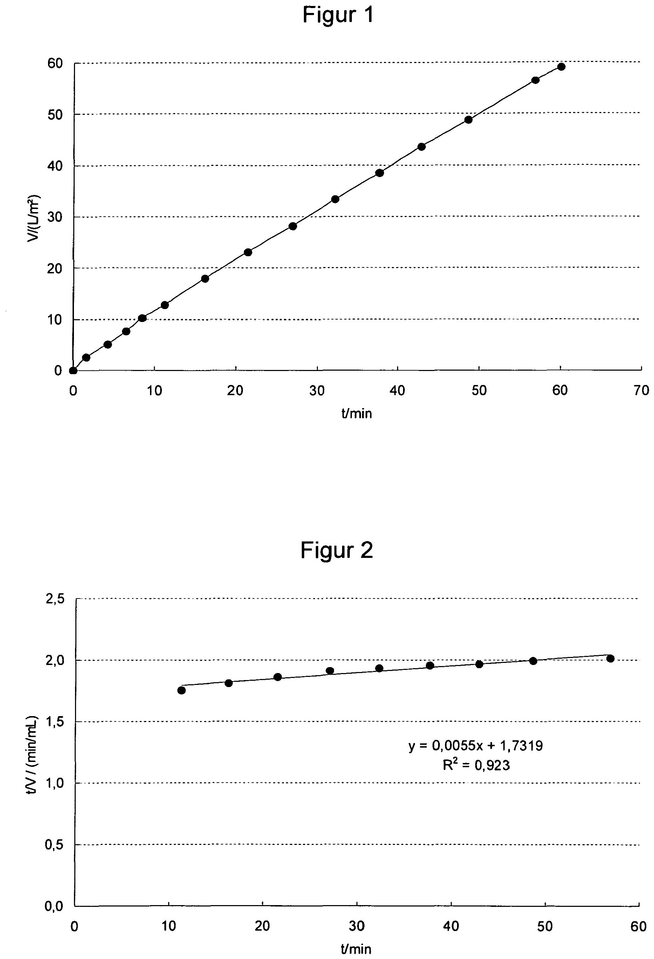

Figur 1 zeigt ein Diagramm des Verlaufs der Filtration des monoklonalen Antikörpers, wobei das filtrierte Volumen, normiert auf 1 m² Fläche, auf der y-Achse und die Zeit der Filtration in Minuten auf der x-Achse aufgetragen ist. Figur 2 zeigt eine Auftragung von $t/V$ gegen $t$ der gleichen Filtration, aus deren Kehrwert der Geradensteigung sich gemäß Gleichung 1 eine Filtrationskapazität $V_{max}$ von 365 L/m² bestimmen lässt.

Vergleichsbeispiel 1: Filtration einer Proteinlösung eines monoklonalen Antikörpers aus der Zelllinie CHO dg44 ST1-C6

Die Filtration von Vergleichsbeispiel 1 wurde analog zu Beispiel 1 durchgeführt, wobei die mikroporöse Polyamid-Membran mit einer Porengröße von 0,2 μm in Schritt (a) durch eine Polyethersulfonmembran, Sartopore® 2 (Porengröße 0,1 μm, Fläche 17,5 cm², Sartorius Stedim Biotech GmbH), ersetzt wurde. Im Schritt (b) wurde das Filtrat aus Schritt (a) normal, d.h. in einer "Dead-End"-Filtration über eine Lage einer virusretentiven Membran, Virosart® CPV Minisart (Fläche 5 cm², Sartorius Stedim Biotech GmbH), bei 1 bar filtriert.

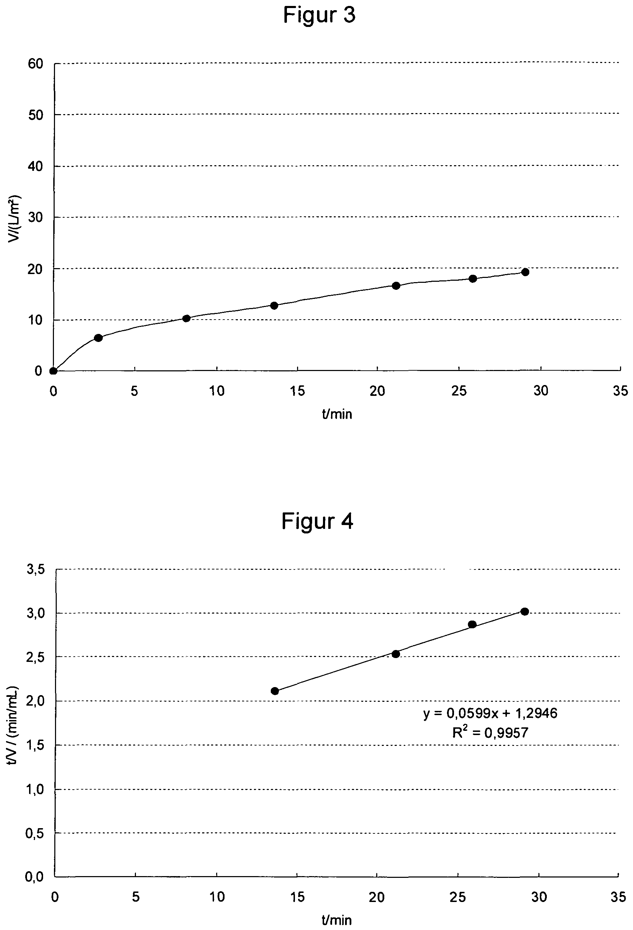

Figur 3 zeigt den Verlauf der Filtration mit dem filtrierten Volumen auf der y-Achse, normiert auf 1 m² Fläche, und der Zeit der Filtration in Minuten auf der x-Achse. Figur 4 zeigt eine Auftragung von $t/V$ gegen $t$ der gleichen Filtration, aus deren Kehrwert der Geradensteigung sich gemäß Gleichung 1 eine Filtrationskapazität $V_{max}$ von 33 L/m² bestimmen lässt. Die Filtrationskapazität beträgt demnach weniger als 10% der Filtrationskapazität unter Verwendung der porösen Polyamid-Oberfläche im Schritt (a) des Beispiels 1.

Beispiel 2: Filtration einer Lösung eines humanen IgG-Proteins

Zur Filtration wurde eine Lösung eines humanen IgG-Proteins (5 %ige Lösung, SeraCare, Katalog Nr. HS-475-1L) verwendet, die mit Phosphat-Puffer, pH 6,6 (Sigma Aldrich, Katalog Nr. P8165), und EDTA (Sigma Aldrich, Katalog Nr. E6758) auf eine Konzentration von 10 g/L Protein und 10 mM EDTA verdünnt wurde. Die Lösung wurde im Schritt (a) durch eine Filtration normal zur äußeren Oberfläche mit einer porösen Polyamid-Oberfläche aus Polyamid 6 in Form einer mikroporösen Membran (Porengröße 0,1 μm, äußeren Fläche 7 cm²) in Kontakt gebracht. Anschließend wurde das Filtrat vom Schritt (a) im Schritt (b) normal über einen doppellagigen Virusfilter Virosart® CPV Minisart (Fläche 5 cm², Sartorius Stedim Biotech GmbH) bei 2 bar filtriert.

Vergleichsbeispiel 2: Filtration einer Lösung eines humanen IgG-Proteins

Vergleichsbeispiel 2 wurde analog zu Beispiel 2 durchgeführt, wobei in Schritt (a) jedoch die poröse Polyamid-Oberfläche aus Beispiel 2 durch eine Polyethersulfonmembran, Sartopore® 2 (Porengröße 0,1 μm, Fläche 17,5 cm², Sartorius Stedim Biotech GmbH), ersetzt wurde.

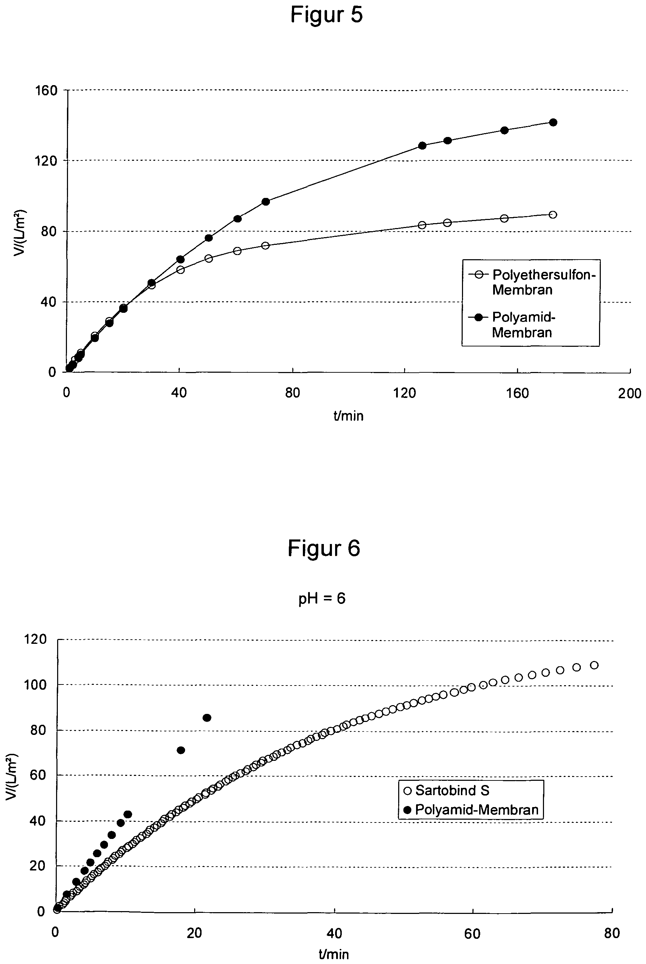

Figur 5 zeigt ein Diagramm des Verlauf der Filtration des humanen IgG-Proteins sowohl von Beispiel 2 (Symbol ●), als auch von Vergleichsbeispiel 2 (Symbol ○), wobei das filtrierte Volumen, normiert auf 1 m² Fläche, auf der y-Achse und die Zeit der Filtration in Minuten auf der x-Achse aufgetragen ist. Die Filtrationskapazität beträgt 109 L/m² für die Filtration unter Verwendung der Polyethersulfonmembran. Mit der Polyamid-Oberfläche als Vorfilter ergibt sich eine Filtrationskapazität von 217 L/m².

Die Verwendung der Polyamid-Oberfläche im ersten Schritt erhöht die Filtrationskapazität bei diesem Medium um etwa den Faktor 2 relativ zur Verwendung einer mikroporösen Polyethersulfonmembran.

Beispiel 3: Untersuchung der pH-Abhängigkeit

Eine Proteinlösung wurde wie in Beispiel 1 beschrieben hergestellt, und die Konzentration auf 10 g/L eingestellt. Der pH-Wert wurde über Änderungen der Pufferzusammensetzung mit Hilfe von Zitronensäure (Sigma Aldrich, Katalog Nr. C2404) und Tris-Base (2-Amino-2-hydroxymethylpropan-1,3-diol, Sigma Aldrich, Katalog Nr. T1503) auf pH = 6,0, beziehungsweise 9,0, eingestellt. Die beiden Proteinlösungen wurden durch Zugabe von Natriumchlorid (Sigma Aldrich, Katalog Nr. S5886) auf eine einheitliche Leitfähigkeit von 15 mS/cm eingestellt. Beide Lösungen wurden über eine Polyethersulfonmembran Sartopore® 2 (Porengröße 0,1 μm) filtriert, um Effekte verschiedener Porengrößen in weiteren Vorfiltrationsschritten auszuschließen. Im Schritt (a) wurden die Proteinlösungen entweder über eine geladene Membran Sartobind® S (äußere Fläche 6 cm$^2$, Sartorius Stedim Biotech GmbH) oder über eine mikroporöse Polyamidmembran (Porengröße 0,1 μm, äußere Fläche 6 cm$^2$) filtriert. Im Schritt (b) wurden die Lösungen über eine Lage eines Parvovirusfilters Virosart® CPV (äußere Fläche 2,5 cm$^2$, Sartorius Stedim Biotech GmbH) und bei einem Differenzdruck von 2 bar filtriert.

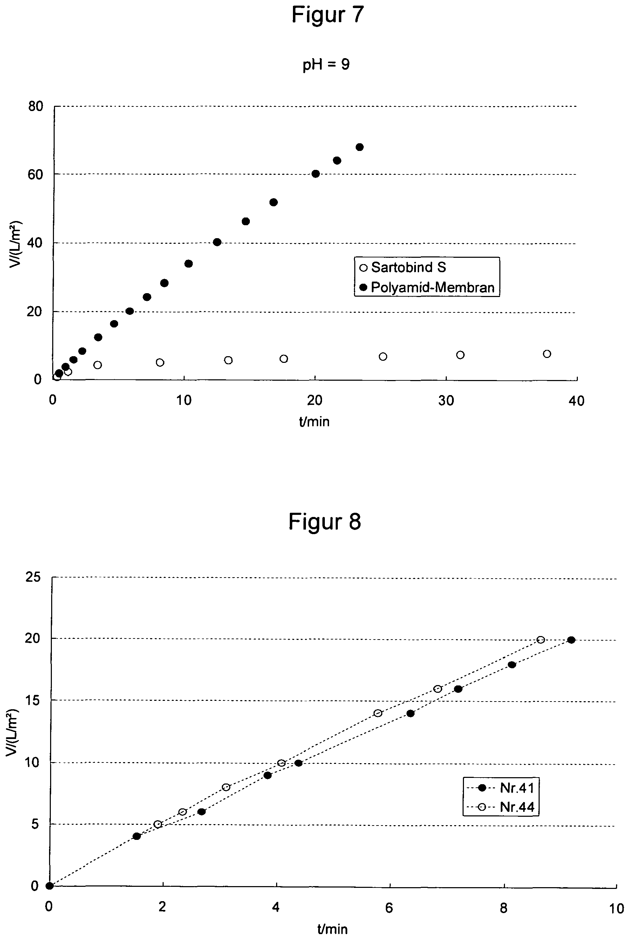

In Figur 6 sind die Filtrationen der Proteinlösung bei pH = 6 sowohl über die poröse Polyamid-Oberfläche in Form einer mikroporösen Polyamidmembran (Symbol ●), als auch über die geladene Membran Sartobind® S (Symbol ○) gemeinsam dargestellt. In Figur 7 sind die Filtrationen der gleichen Proteinlösung bei pH = 9 dargestellt. In beiden Figuren sind das filtrierte Volumen, normiert auf 1 m$^2$ Fläche, auf der y-Achse und die Zeit der Filtration in Minuten auf der x-Achse aufgetragen.

Der Unterschied zwischen der Sartobind® S Membran und der Polyamidmembran als erstem Schritt vor der Filtration über eine virusretentive Membran ist deutlich zu erkennen. In der hier aufgeführten Reihe von Filtrationsversuchen wird verdeutlicht, dass die Erhöhung der Filtrationskapazität einer virusretentiven Membran unter Verwendung von Polyamidoberflächen bei verschiedenen pH-Werten der Lösung gleichermaßen auftritt, wobei der bekannte Prozess der Verwendung von geladenen Membranen eine deutliche pH-Abhängigkeit zeigt. Bei pH = 6 ist der Abstand der Filtrationskapazität des Virenfilters zwischen der Polyamid-Oberfläche mit $V_{max}$ = 550 L/m$^2$ und der geladenen Membran mit $V_{max}$ = 200 L/m$^2$ noch relativ gering. Bei pH = 9 zeigt sich ein deutlich größerer Abstand, hier bricht die Filtrationskapazität des Virenfilters hinter der geladenen Membran stark ein; mit der geladenen Membran werden nur $V_{max}$ = 8 L/m$^2$ erreicht. Bei Verwendung der Polyamidoberfläche vor dem Virusfilter werden hingegen $V_{max}$ = 260 L/m$^2$ erreicht.

Beispiel 4: Abreicherung von Biopolymer-Aggregaten und Viren

Beispiel 4 zeigt eine Wiederholung des Beispiels 1 in einer leicht abgewandelten Form und demonstriert zusätzlich die Abreicherung der Viren durch das erfindungsgemäße Verfahren.

Eine Proteinlösung eines monoklonalen Antikörpers aus der Zelllinie CHO dg44 ST1-C6 wurde wie in Beispiel 1 beschrieben vorbereitet. Vor dem Einsatz als Filtrationsmedium wurde die Proteinlösung jedoch mit einer Lösung von Bakteriophagen des Typs PP7, einem anerkannten Modellsystem für kleine, nicht umhüllte Viren versetzt, so dass die Lösung 10 g/L Protein und 4,5 · 10$^7$ pfu/mL ("plaque forming units") des Bakteriophagen enthielt. Die Filtration wurde analog zu Beispiel 1, jedoch bei 2,0 bar Differenzdruck zweimal durchgeführt. Als Vorfilter wurde dabei jeweils ein Vorfilter des Typs Polyamid aus Beispiel 1 verwendet. Als Hauptfilter wurden aus einer Charge von 200 Virenfiltern Virosart® CPV zufällig zwei Virenfilter ausgewählt (Nr. 41 (Symbol ●) bzw. Nr. 44 (Symbol ○)) und für die Filtration verwendet. Bei jeweils 10 L/m$^2$ und 20 L/m$^2$ wurde eine Probe entnommen und der Titer der Bakteriophagen nach einem Plaque Essay bestimmt. Die Ergebnisse der Titerbestimmung sind in Tabelle 1 dargestellt.

Tabelle 1:

| Nr. Hauptfilter* | Filtrat Nr. | filtriertes Volumen [L/m$^2$] | Filtrationszeit | Upstream Titer [pfu/mL] | Downstream Titer [pfu/mL] | Log10 ** |
|---|---|---|---|---|---|---|
| 41 | 1.1 | 10 | 4 min 22 s | 4,5 · 10$^7$ | 2,0 · 10$^1$ | 6,3 |
| 41 | 1.2 | 20 | 9 min 10 s | 4,5 · 10$^7$ | 2,0 · 10$^1$ | 6,3 |
| 44 | 2.1 | 10 | 4 min 4 s | 4,5 · 10$^7$ | 2,0 · 10$^1$ | 6,3 |
| 44 | 2.2 | 20 | 8 min 37 s | 4,5 · 10$^7$ | 2,0 · 10$^1$ | 6,3 |

* Hauptfilter = Virenfilter Virosart CPV
** Der Wert 6,3 als Eintrag in der Spalte "Log10" gibt an, dass der Bakteriophagentiter um 10$^{6.3}$ pfu reduziert wurde.

Der Verlauf der Filtration ist in Figur 8 dargestellt. Aus einer Auftragung gemäß Gleichung 1 ergeben sich die Filtrationskapazitäten zu 286 L/m$^2$ für Hauptfilter Nr. 41 und 130 L/m$^2$ für Hauptfilter Nr. 44. Wegen der erfindungsgemäß gering ausgeprägten Verblockung zum Endpunkt der Filtration ist die Extrapolation auf die maximale Kapazität mit einer gewissen Schwankungsbreite verbunden.

**Patentansprüche**

1. Verfahren zur Abtrennung von Biopolymer-Aggregaten und Parvoviren aus einem Fluid, umfassend die Schritte:

(a) Filtrieren des Fluids, welches die Biopolymer-Aggregate und Parvoviren enthält, durch einen porösen, Polyamid umfassenden Formkörper, dessen innere und äußere Oberflächen die gleichen chemischen und physikalischen Eigenschaften aufweisen wie die von den Oberflächen eingeschlossene Matrix des Formkörpers, wobei selektiv die Biopolymer-Aggregate adsorptiv aus dem Fluid abgereichert werden, während die Parvoviren durch den Formkörper permeieren; und

(b) Filtrieren des Fluids, das durch Filtration im Schritt (a) erhalten wurde, durch mindestens eine Membran mit einem Molekulargewichtsausschlußbereich von 100 bis 1.000 kD, wobei der Gehalt an Parvoviren in dem Fluid gegenüber dem Gehalt an Parvoviren vor Durchführen von Schritt (a) um mindestens 99,9 % reduziert wird;

wobei der poröse, Polyamid umfassende Formkörper eine mikroporöse Membran ist und aus einem aliphatischen und/oder aromatischen Polyamid mit höchstens fünf verschiedenen Wiederholungseinheiten besteht, wobei die Membran, die im Schritt (b) verwendet wird, aus Polyethersulfon, Polysulfon oder Gemischen davon besteht, wobei die Biopolymer-Aggregate aus der Gruppe der Dimere, Trimere oder Multimere von Peptiden, Proteinen, Nukleinsäuren oder Gemischen davon ausgewählt sind und wobei der pH-Wert des Fluids während der Schritte (a) und (b) zwischen 5 und 9 beträgt.

2. Verfahren nach Anspruch 1, wobei der poröse, Polyamid umfassende Formkörper Polyamid 6 und/oder Polyamid 6.6 umfasst.

3. Verfahren nach Anspruch 1 oder 2, wobei das Fluid ein Blutplasmaprodukt, eine aus einer Zellkultur gewonnene Proteinlösung, eine aus einer Extraktion von tierischen oder pflanzlichen Produkten gewonnene Proteinlösung oder eine aus Mikroorganismen gewonnene Proteinlösung umfasst.

**Claims**

1. A method for removing biopolymer aggregates and parvoviruses from a fluid, comprising the steps of:

(a) filtering the fluid containing the biopolymer aggregates and parvoviruses through a porous, polyamide-comprising shaped body, the internal and external surfaces of which have the same chemical and physical properties as the shaped-body matrix which is enclosed by the surfaces, wherein the biopolymer aggregates are selectively depleted from the fluid by adsorption, whereas the parvoviruses permeate through the shaped body; and

(b) filtering the fluid from step (a) through at least one membrane having a molecular weight cut-off of from 100 to 1,000 kD, wherein the content of parvoviruses in the fluid is reduced by at least 99.9% with respect to the content of parvoviruses prior to carrying out step (a) ;

wherein the porous, polyamide-comprising shaped body comprises a microporous membrane and consists of an aliphatic and/or aromatic polyamide having no more than five different repeat units, wherein the membrane used in step (b) consists of polyethersulfone, polysulfone, or mixtures thereof, wherein the biopolymer aggregates are selected from the group of the dimers, trimers or multimers of peptides, proteins, nucleic acids or mixtures thereof and wherein the pH of the fluid during steps (a) and (b) is between 5 and 9.

2. The method according to claim 1, wherein the porous, polyamide-comprising shaped body comprises polyamide 6 and/or polyamide 6.6

3. The method according to claim 1 or 2, wherein the fluid comprises a blood plasma product, a protein solution obtained from a cell culture, a protein solution obtained from extraction of animal or plant products, or a protein solution obtained from microorganisms.

**Revendications**

1. Procédé pour la séparation d'agrégats de biopolymère et de parvovirus d'un fluide, comprenant les étapes consistant à :

   (a) filtrer le fluide qui contient les agrégats de biopolymère et les parvovirus à travers un corps moulé poreux, comprenant du polyamide, dont les surfaces intérieures et extérieures présentent les mêmes propriétés chimiques et physiques que la matrice du corps moulé enfermée par les surfaces, dans lequel les agrégats de biopolymère sont sélectivement appauvris du fluide par adsorption tandis que les parvovirus passent par perméation à travers le corps moulé ; et
   (b) filtrer le fluide, qui a été obtenu par filtration à l'étape (a), à travers au moins une membrane ayant une plage d'exclusion de poids moléculaire de 100 à 1 000 kD, dans lequel la teneur en parvovirus dans le fluide par rapport à la teneur en parvovirus avant la réalisation de l'étape (a) est réduite d'au moins 99,9 % ;

   dans lequel le corps moulé poreux comprenant du polyamide est une membrane microporeuse et se compose d'un polyamide aliphatique et/ou aromatique comportant au maximum cinq motifs répétitifs différents,
   dans lequel la membrane utilisée à l'étape (b) se compose de polyéther-sulfone, polysulfone ou de mélanges de celles-ci,
   dans lequel les agrégats de biopolymère sont choisis dans le groupe des dimères, trimères ou multimères de peptides, de protéines, d'acides nucléiques ou de mélanges de ceux-ci, et
   dans lequel le pH du fluide pendant les étapes (a) et (b) est compris entre 5 et 9.

2. Procédé selon la revendication 1, dans lequel le corps moulé poreux comprenant du polyamide comprend du polyamide 6 et/ou du polyamide 6.6.

3. Procédé selon la revendication 1 ou 2, dans lequel le fluide est un produit de plasma sanguin, une solution de protéine obtenue à partir d'une culture cellulaire, une solution de protéine obtenue à partir d'une extraction de produits animaux ou végétaux ou une solution de protéine obtenue à partir de micro-organismes.

## Figur 1

## Figur 2

$$y = 0,0055x + 1,7319$$
$$R^2 = 0,923$$

## Figur 3

## Figur 4

$$y = 0{,}0599x + 1{,}2946$$
$$R^2 = 0{,}9957$$

# Figur 5

# Figur 6

pH = 6

# Figur 7

pH = 9

# Figur 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7118675 B2
- EP 1624950 B1
- US 5531899 A
- US 5093197 A
- US 7465397 B2
- US 20090326211 A1
- WO 2010098867 A1
- JP 2005145852 A

**In der Beschreibung aufgeführte Nicht-Patentliteratur**

- Increasing Parvovirus Filter Throughput of Monoclonal Antibodies Using Ion Exchange Membrane Adsorptive Pre-Filtration. **A. BROWN.** Bioseparations and Downstream Processing, Biotechnology and Bioengineering. Wiley Periodicals, Inc, 2010
- **HO.** *Journal of Colloid and Interface Science,* 2000, vol. 232, 389-399